# EUROPEAN PATENT SPECIFICATION

(11) **EP 2 499 915 B1**
(45) Date of publication and mention of the grant of the patent: **04.10.2017**
(21) Application number: 10829923.1
(22) Date of filing: 09.11.2010
(51) Int. Cl.: A01N 59/06, A01N 31/02, A01N 37/36, A01P 1/00, A23B 4/027, A23L 3/358, A61K 31/045, A61K 31/19, A61K 33/08, A01N 31/04, A23L 17/00, A61K 45/06, A23B 4/24, A23L 3/3463, A23B 4/20

(54) **NON THERAPEUTIC USE OF A VIRUS-INACTIVATING AGENT**
NICHT THERAPEUTISCHE VERWENDUNG EINES VIRENDEAKTIVIERUNGSMITTELS
UTILISATION NON THÉRAPEUTIQUES D'UN AGENT D'INACTIVATION VIRALE

(30) Priority: 10.11.2009 JP 2009257384
(43) Date of publication of application: 19.09.2012
(73) Proprietor: Kawakami Co., Ltd., Osaka 553-0005 (JP)
(72) Inventor: KAWAKAMI, Hiroo, Nishinomiya-shi Hyogo 662-0051 (JP); KOTANI, Hirohide, deceased (JP); YAMASHITA, Yasuharu, Osaka-shi Osaka 553-0005 (JP)
(74) Representative: Kloiber, Thomas
(86) International application number: PCT/JP2010/069919
(87) International publication number: WO 2011/058964

(56) References cited:
- EP-A1- 1 366 677
- EP-A1- 2 241 321
- EP-A1- 2 446 755
- WO-A1-2005/013695
- WO-A1-2009/078447
- WO-A1-2009/084211
- WO-A1-2009/104670
- WO-A1-2010/044194
- JP-A- 2001 226 210
- JP-A- 2011 012 000
- DATABASE WPI Thomson Scientific, London, GB; AN 2007-801455 XP002708133, "Preserving or transporting live fish and shellfish, involves placing live fish and shellfish in seawater and adding glycine to seawater", & JP 2007 259766 A (CHIBA SUISAN KK) 11 October 2007 (2007-10-11)
- DATABASE WPI Thomson Scientific, London, GB; AN 1978-43205A XP002708134, "Preservation of paste type food e.g. fish or artificial meat-by making the paste weakly alkaline then adding glycine and/or L-serine and alkali metal sal of organic acid", & JP 5 350361 A (OGAWA) 8 May 1978 (1978-05-08)
- DATABASE WPI 24 July 2003 (2003-07-24) Thomson Scientific, London, GB; AN 2003-608014 XP002708368, "Preventing browning or blackening of peeled shellfish by treating with aqueous alkali, then washing off or neutralizing the alkali to prolong storage life", & WO 03/059074 A1 (HIRAOKA) 24 July 2003 (2003-07-24)

## Description

### BACKGROUND OF THE INVENTION

### 1. Field of the Invention

The present invention relates to the non therapeutic use of a virus-inactivating agent having inactivating effects against norovirus and influenza A virus.

### 2. Description of the Related Art

Since previously, compositions having an alcohol as a main agent have been widely used as microbe eradicating agents, and further as microbe eradicating agents having an inactivating effect against norovirus, a composition in which a grapefruit seed extract and phytic acid are blended with brewer' s alcohol (Patent Document 1), a composition in which an alkaline substance and a surfactant are blended with ethanol (Patent Document 2), and a composition in which an organic acid salt and a eucalyptus extract are blended with ethanol (Patent Document 3), have been disclosed.

Meanwhile recently, it has been disclosed that calcinated shell calcium, calcium oxide, and calcium hydroxide as a hydrate of calcium oxide, have antimicrobial properties (Patent Document 4), and a microbe suppressing agent for foods in which an organic acid salt is blended with calcinated shell calcium (Patent Document 5), a microbe eradicating agent for foods in which a polyvalent alcohol fatty acid ester and ethanol are blended with calcinated calcium (Patent Document 6) have been disclosed. However, an inactivating effect of calcinated shell calcium, etc. against norovirus was unknown.

Patent Document 1: Japanese Published Unexamined Patent Application: No. 2007-320924
Patent Document 2: Japanese Published Unexamined Patent Application: No. 2008-189645
Patent Document 3: Japanese Published Unexamined Patent Application: No. 2009-179577
Patent Document 4: Japanese Published Unexamined Patent Application: No. H11-222796
Patent Document 5: Japanese Published Unexamined Patent Application: No. H11-290044
Patent Document 6: Japanese Published Unexamined Patent Application: No. 2002-272434

In EP 2 241 321 A1 a hydroxy radical generation method and anti-viral material utilizing hydroxy radical generated by the method are disclosed.

WO 2009/104670 A1 discloses an antibacterial antiviral agent which is effective against influenza virus type A and particularly preferred to apply to Caliciviridae norovirus and which may be subjected to water or be used in suspension in a dispersion medium. Further the agent is prepared by blending in calcium hydroxide, calcium oxide and/or calcinated calcium. WO 2009/078447 A discloses that ethanol and sodium lactate may be contained as an additive in an antiviral agent. There is no description about what effect such additives exert as auxiliary agents.

WO 2009/084211 A1 discloses a disinfectant composition which expresses an effect on a virus such as norovirus, however, does not comprise any calcium containing compounds.

The present invention has been made in view of the above circumstances and objects thereof are to elucidate virus-inactivating effects of calcinated calcium, and provide a novel virus-inactivating agent having, inparticular, a norovirus-inactivating effect.

As a result of conducting diligent research, the present inventors found that an aqueous solution or aqueous dispersion in which calcium hydroxide is blended not only has a norovirus-inactivating effect but also has an influenza A virus-inactivating effect, and have thereby come to complete the present invention.

That is, in gist, the present invention provides the following.
[1] The use of an agent for inactivating norovirus and influenza A virus, wherein the agent is an aqueous solution or aqueous dispersion prepared by blending in calcium hydroxide, calcium oxide and/or calcinated calcium and by further blending in ethanol and sodium lactate, wherein a total blending proportion of the blended components among calcium hydroxide, calcium oxide, and calcinated calcium is 5 to 30 weight %, and a blending proportion of sodium lactate is 1 to 30 weight %.
[2] The virus-inactivating agent according to [1] where an average particle diameter of calcium hydroxide, calcium oxide, and calcinated calcium is 0.1 to 10µm.

By the present invention, a virus-inactivating agent having a norovirus-inactivating effect and an influenza A virus-inactivating effect is provided.

### BRIEF DESCRIPTION OF THE DRAWINGS

FIG. 1 is an electrophoretogram of PCR products;
FIG. 2 is an electrophoretogram of PCR products;

### DETAILED DESCRIPTION OF THE PREFERRED EMBODIMENTS

The virus inactivating agent used in the present invention is an aqueous solution or aqueous dispersion obtained by blending calcium hydroxide with water and has a norovirus-inactivating effect, an influenza A virus-inactivating effect, and a feline calicivirus-inactivating effect.

As blended components of the present invention, one or more of a component that reacts with water to produce calcium hydroxide, for example, calcium oxide, calcinated calcium, may be used in place of calcium hydroxide or in addition to calcium hydroxide. Hereinafter in the present specification, calcium hydroxide and components, such as calcium oxide, calcinated calcium, that react with water to produce calcium hydroxide, shall be referred to collectively as "virus-inactivating components."

Among the virus-inactivating components, calcinated calcium is composed mainly of calcium oxide and is obtained by subjecting animal-derived calcium which are composed of calcium carbonate before calcination, such as oyster shells, scallop shells, surf clam shells, eggshells, or coral shells, to calcination or ohmic heating at a temperature of no less than 600°C and preferably 900 to 1200°C for approximately 15 to 60 minutes. Preferably, a saturated aqueous solution of the calcinated calcium obtained has a pH in a range of 11 to 13. As the calcinated calcium, that which complies with food additive standards is normally used.

An average particle diameter of the virus-inactivating components is not restricted in particular and is normally 0.1 to 10µm. Also, a blending proportion of the virus-inactivating components in the virus-inactivating agent is not restricted in particular and is normally 0.01 to 30 weight %.

With the present invention, as other blended components besides the virus-inactivating components, for example, ethanol or an organic acid salt may be blended to increase the virus-inactivating effect. As the ethanol, that which complies with food additive standards is normally preferable. A blending proportion of the ethanol in the virus-inactivating agent is not restricted in particular, and the ethanol is normally blended in a range of 5 to 30 weight %.

Examples of the organic acid salt include citrates, lactates, malates, tartrates, oxalates, acetates, formates, succinates, fumarates, gluconates, and these may be used solely or in suitable combination of two or more thereof. Examples of the salt include an alkali metal salt. Among the above, lactates are preferable and sodium lactates are more preferable. A blending proportion of the organic acid salt in the virus-inactivating agent is not restricted in particular, and the organic acid salt is normally blended in a range of 1 to 30 weight %.

In the virus-inactivating agent in addition to the above-described components, known components that can be blended in a microbe eradicating agent may be contained at suitable amounts in ranges at which the effects of the present invention are not impaired. Examples of such components include antiseptics, antioxidants, surfactants, pigments, fragrances, dyes.

A pH of the virus-inactivating agent is preferably 11 to 14 and more preferably 11 to 13 from the standpoint of the virus-inactivating effect.

The virus-inactivating agent used in the present invention is especially significant in the norovirus-inactivating effect and the influenza A virus-inactivating effect. In accordance with concentrations of the above-described blended components, the present invention may be used as an aqueous solution type in which the respective components are practically dissolved in water or as an aqueous dispersion type in which a portion of the components is dissolved in water and a portion of the components is dispersed in water. Although the virus-inactivating agent may be used as it is without diluting with water before use, in a case of using an aqueous dispersion type, it is preferable to dilute it with water before use. In employing any usage, such as using an aqueous solution type or an aqueous dispersion type as it is or diluting either with water before use, the blending proportion of the virus-inactivating components in the virus-inactivating agent is preferably such that an actual usage concentration of the components is 0.01 to 5 weight %.

In a case of using an aqueous solution type, the virus-inactivating agent may be filled in a spray container and used by spraying onto a worker's hands, arms, and other skin portions, knives, pots, and other cooking utensils, plates, containers, and other eating utensils, fresh fish and shellfish, vegetables, meats, and other fresh foods, processed foods, to which the abovementioned viruses may be attached, in a food factory, restaurant kitchen, or household kitchen. In a case of using an aqueous dispersion type, the virus-inactivating agent may be diluted with water before use and may be used by immersing therein the portable objects among the applicable objects included in the examples above.

### EXAMPLES

### 1. Verification of inactivating effect against feline calicivirus

### A. Outline of the test

An aqueous dispersion (the present invention product) of the formulation indicated below was prepared as a test substance by blending calcium hydroxide, ethanol, and sodium lactate and using water as the remaining portion, and a verification test concerning the inactivating effect of the invention product against feline calicivirus was performed at Japan Food Research Laboratories. That is, norovirus itself was not cultured with stability and thus in the present test, feline calicivirus, which was closely related to norovirus in morphological characteristics and genome structure, was used as an alternative virus. Details of the test are as indicated below.

### B. Test method

### 1) Test substance: Aqueous dispersion (the present invention product)

| | |
|---|---|
| Calcium hydroxide | 12.8 weight % |
| Ethanol | 19.7 weight % |
| Sodium lactate | 9.9 weight % |
| Water | 57.6 weight % |

### 2) Test virus

Feline calicivirus F-9 ATCC VR-782 (feline calicivirus)

### 3) Cells used

CRFK cells (Dainippon Pharmaceutical Co., Ltd.)

### 4) Media used

### (1) Cell growth medium

Eagle MEM "Nissui" (1) (Nissui Pharmaceutical Co. Ltd.) with fetal bovine serum added at 10%.

### (2) Cell maintenance medium

Eagle MEM "Nissui" (1) (Nissui Pharmaceutical Co. Ltd.) with fetal bovine serum added at 2%.

### 5) Preparation of virus suspension

### (1) Culturing of cells

The cells used were monolayer-cultured in a tissue culture flask using the cell growth medium.

### (2) Inoculation of virus

After monolayer-culturing, the cell growth medium was removed from the inside of the flask and the test virus was inoculated. The cell maintenance medium was then added and culturing was performed for 1 to 5 days in a carbon dioxide gas incubator (CO₂ concentration: 5%) set at 37°C.

### (3) Preparation of virus suspension

After culturing, the cell morphology was observed using an inverted phase contrast microscope to confirm that morphological changes of cells (cytopathic effect) occurred. The culture solution was then centrifuged (3,000r/min, for 10 minutes) and the resulting supernatant liquid was used as the virus suspension.

### 6) Test procedure

Using purified water, a 2% suspension of the test substance was prepared as a test liquid. After adding 0.1ml of the virus suspension to 1ml of the test liquid and mixing, the mixture was storedunder room temperature while shaking at 80 to 90r/min, and 6 and 24 hours later, the test liquid was diluted by 100 times using the cell maintenance medium.

As a control test liquid, the purified water was also subject to the test in the same manner. However, with the purified water, measurement was also made at the start time.

### 7) Measurement of virus infectivity titer

The cells used were monolayer-cultured using the cell growth medium in a tissue culture microplate (96-well) and thereafter, the cell growth medium was removed and 0.1ml of the cell maintenance medium was added to each well. 0.1ml of the diluted test liquid was then inoculated into each of four wells and culturing was performed for 4 to 7 days in a carbon dioxide gas incubator (CO₂ concentration: 5%) set at 37°C. After culturing, an inverted phase contrast microscope was used to observe whether or not morphological changes of cells (cytopathic effect) occurred, and a median tissue culture infectious dose (TCID₅₀) was calculated by the Reed-Muenchmethod and converted to a virus infectivity titer per 1ml of test liquid. The results are shown in Table 1.

**[Table 1]**

| Test virus | Subject | log TCID₅₀/ml ^{*1} | | |
|---|---|---|---|---|
| | | Start time | 6 hours later | 24 hours later |
| Feline calicivirus | Test substance | 8.5 | <2.5 | <2.5 |
| | Control | 8.5 | 8.2 | 7.8 |

| | | | | |
|---|---|---|---|---|
| TCID₅₀: median tissue culture infectious dose *1: Logarithmic value of TCID₅₀ per 1ml of the test liquid Start time: The TCID₅₀ of the control which was measured immediately after the onset of action was set as that was measured at the start time. Control: Purified water Action conditions : Storage while shaking under room temperature <2.5: Not detected | | | | |

Table 1 shows that the invention product completely inactivated feline calicivirus of 3.1×10⁸TCID₅₀/ml within 6 hours.

### 2. Verification of inactivating effect against norovirus

(1) An aqueous solution (invention product) of the formulation indicated below was prepared as a test substance by blending calcium hydroxide, ethanol, and sodium lactate and using water as the remaining portion, and a verification test concerning the inactivating effect of the invention product against norovirus was performed at the Food Analysis Center of Visionbio Corporation.

### <Test substance: Aqueous solution (invention product)>

| | |
|---|---|
| Calcium hydroxide | 0.26 weight % |
| Ethanol | 10 weight % |
| Sodium lactate | 5 weight % |
| Water | 84.74 weight % |

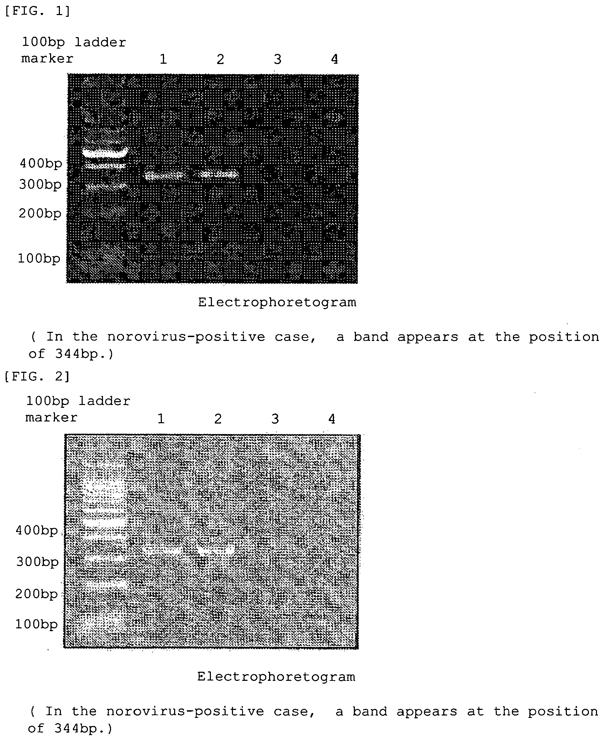

A norovirus detection test was performed in accordance with the "Norovirus Detection Method" (latest revision: Food Safety Monitor Issue Report No. 0514004, May 14, 2007) published by the Japanese Ministry of Health, Labor, and Welfare. Fecal norovirus (belonging to NV genogroup II) was used as the norovirus. As the experimental procedure, each of the four samples indicated below was stirred by a vortex mixer, RNA extraction was started after letting stand for 10 minutes, DNase treatment and RT-PCR reaction (reverse transcriptase polymerase chain reaction) were performed, and the PCR product obtained was subject to 2.5% agarose gel electrophoresis. After the end of electrophoresis, gel staining and photography were performed and bands were checked. In the RT-PCR reaction, G2-SKF/G2-SKR (amplification fragment; 344bp) and G2-SKF/G2AL-SKR (amplification fragment; 344bp) were used as primers for genogroup (G) II detection. An electrophoretogram of the PCR products is shown in FIG. 1.

### <Samples>

1: Sample without anything added to 500µl of a norovirus suspension (control group 1)
2: Sample with 400µl of purified water added to 100µl of the norovirus suspension (control group 2)
3: Sample with 400µl of the invention product added to 100µl of the norovirus suspension (invention product added group 1)
4: Same sample as 3 above (invention product added group 2)

In the present verification test, a band appears at the 344bp position in the case of a norovirus-positive result. FIG. 1 shows that whereas a band was not seen and the norovirus gene was thus not detected in each of Samples 3 and 4 to which the invention product was added, the band of the norovirus gene was detected in each of Samples 1 and 2 to which the invention product was not added. It was thus confirmed that under the conditions of the present verification test, the invention product has an inactivating effect against norovirus.
(2) Besides using an aqueous solution (invention product) that was obtained by blending calcium hydroxide with water and not blending in ethanol and sodium lactate in accordance with the formulation shown below as a test substance and stirring each of the four samples indicated below with the vortex mixer and starting RNA extraction after letting stand for 30 minutes, a verification test concerning an inactivation effect against norovirus was performed by the same method as that of (1) above. An electrophoretogram of the PCR products is shown in FIG. 2.

### <Test substance: Aqueous solution (invention product)>

| | |
|---|---|
| Calcium hydroxide | 0.26 weight % |
| Water | 99.74 weight % |

### <Samples>

1: Sample without anything added to 500µl of the norovirus suspension (control group 1)
2: Sample with 400µl of purified water added to 100µl of the norovirus suspension (control group 2)
3: Sample with 400µl of the invention product added to 100µl of the norovirus suspension (invention product added group 1)
4: Same sample as 3 above (invention product added group 2)

FIG. 2 shows that whereas a band was not seen and the norovirus gene was thus not detected in each of Samples 3 and 4 to which the invention product was added, the band of the norovirus gene was detected in each of Samples 1 and 2 to which the invention product was not added. It was thus confirmed that under the conditions of the present verification test, the invention product has an inactivating effect against norovirus.

In comparing the experimental conditions of (1) and (2) above, whereas in (1), the time of treatment against norovirus by the invention product (in which calcium hydroxide, ethanol, and sodium lactate were blended and water was used as the remaining portion) was 10 minutes, in (2), the time of treatment against norovirus by the invention product (in which calcium hydroxide was blended and water was used as the remaining portion) was 30 minutes. And as shown in FIGS. 1 and 2, the band of the norovirus gene was not detected in any of Samples 3 and 4 of (1) and Samples 3 and 4 of (2). It is considered that the time of treatment against norovirus by the invention product is correlated with the level of virus-inactivating effect, and from the results of (1) and (2) above, it is considered that the invention product of (1) is better in terms of virus-inactivating effect than the invention product of (2).

### 3. Verification of inactivating effect against influenza A virus

### A. Outline of the test

The same test substance as that of (1) of "2. Verification of inactivating effect against norovirus" described above was used in performing a verification test concerning the inactivating effect of the invention product against influenza A virus at Japan Food Research Laboratories. Details of the test are as indicated below.

### B. Test method

### 1) Test substance: Aqueous solution (invention product)

| | |
|---|---|
| Calcium hydroxide | 0.26 weight % |
| Ethanol | 10 weight % |
| Sodium lactate | 5 weight % |
| Water | 84.74 weight % |

### 2) Test virus

Influenza A virus (H1N1)

### 3) Cells used

MDCK (NBL-2) cells ATCC CCL-34 strain (Dainippon Pharmaceutical Co., Ltd.)

### 4) Media used

### (1) Cell growth medium

| | |
|---|---|
| Eagle MEM "Nissui" (1) (Nissui Pharmaceutical Co. Ltd.) | 1,000ml |
| 10% NaHCO₃ | 14ml |
| L-glutamine (30g/l) | 9.8ml |
| 100 × vitamin solution for MEM | 30ml |
| 10% albumin | 20ml |
| 0.25% trypsin | 20ml |

### 5) Preparation of virus suspension

### (1) Culturing of cells

The cells used were monolayer cultured in a tissue culture flask using the cell growth medium.

### (2) Inoculation of virus

After monolayer culturing, the cell growth medium was removed from inside the flask and the test virus was inoculated. The cell maintenance medium was then added and culturing was performed for 1 to 5 days in a carbon dioxide gas incubator (CO₂ concentration: 5%) set at 37°C.

### (3) Preparation of virus suspension

After culturing, the cell morphology was observed using an inverted phase contrast microscope to confirm that morphological changes of cells (cytopathic effect) occurred. The culture solution was then centrifuged (3,000r/min, for 10 minutes) and the resulting supernatant liquid was used as the virus suspension.

### 6) Test procedure

0.1ml of the virus suspension was added to and mixed with 1ml of the test substance to prepare an action solution. The action solution was left to act under room temperature, and 30 seconds, 1 minute, and 5 minutes later, it was diluted by 100 times using the cell maintenance medium. As a control, the purified water was also subject to the test in the same manner and subject to measurement at the start time and at 5 minutes later.

### 7) Measurement of virus infectivity titer

The cells used were monolayer-cultured using the cell growth medium in a tissue culture microplate (96-well) and thereafter, the cell growth medium was removed and 1.1ml of the cell maintenance medium was added to each well. 0.1ml of the diluted action solution was then inoculated into each of four wells and culturing was performed for 4 to 7 days in a carbon dioxide gas incubator (CO₂ concentration: 5%) set at 37°C. After culturing, an inverted phase contrast microscope was used to observe whether or not morphological changes of cells (cytopathic effect) occurred, and a median tissue culture infectious dose (TCID₅₀) was calculated by the Reed-Muenchmethod and converted to a virus infectivity titer per 1ml of action solution. The results are shown in Table 2.

**[Table 2]**

| Test virus | Subject | log TCID₅₀/ml^{*} | | | |
|---|---|---|---|---|---|
| | | Start time | 30 seconds later | 1 minute later | 5 minutes later |
| Influenza A virus | Test substance | 7.3 | 3.5 | <2.5 | <2.5 |
| | Control | 7.3 | *** | *** | 7.7 |

| | | | | | |
|---|---|---|---|---|---|
| TCID₅₀: median tissue culture infectious dose *: Logarithmic value of TCID₅₀ per 1ml of the test liquid Start time: The TCID₅₀ of the control which was measured immediately after the onset of action was set as that was measured at the start time. Control: Purified water Action conditions: Room temperature <2.5: Not detected ***: Test not performed | | | | | |

Table 2 shows that the invention product completely inactivated influenza A virus of 2.0 x 10⁷TCID₅₀/ml within 1 minute.

It is presumed that the sterilizing activity enhancement effect of sodium lactate is due to improvement of solubility of calcium hydroxide in the composition. That is, it is generally considered that the calcinated calcium reacts with the water in the composition and is presented as calcium hydroxide and that the calcium hydroxide exhibits the sterilization effect. The solubility of calcium hydroxide in water is low and although it is thus considered as not exhibiting its inherent sterilization effect, the above results seem to suggest that the solubility of the produced calcium hydroxide was improved by the addition of sodium lactate.

The virus-inactivating agent according to the present invention is widely usable as a virus-inactivating agent applicable not only to foods per se but also to cooking utensils, cooking equipment, and other food manufacturing equipment.

## Claims

1. The non therapeutic use of an agent for inactivating norovirus and influenza A virus, wherein the agent is
in an aqueous solution or aqueous dispersion
prepared by blending in calcium hydroxide, calcium oxide and/or calcinated calcium and
by further blending in ethanol and sodium lactate,
wherein a total blending proportion of the blended components among calcium hydroxide, calcium oxide, and calcinated calcium is 0.01 to 30 weight %,
a blending protion of ethanol is 5 to 30 weight %, and
a blending proportion of sodium lactate is 1 to 30 weight %.

2. The virus-inactivating agent according to claim 1, wherein an average particle diameter of calcium hydroxide, calcium oxide, and calcinated calcium is 0.1 to 10 µm.

## Patentansprüche

1. Die nicht therapeutische Verwendung eines Mittels zur Inaktivierung von Norovirus und Influenza-A-Virus, wobei das Mittel
in wässriger Lösung oder wässriger Dispersion ist,
durch Mischen in Calciumhydroxid, Calciumoxid und/oder calciniertem Calcium und
durch weiteres Mischen in Ethanol und Natriumlactat hergestellt ist,
wobei ein Gesamtmischungsanteil der gemischten Komponenten aus Calciumhydroxid, Calciumoxid und calciniertem Calcium 0,01 bis 30 Gew.-% ist,
ein Mischungsanteil von Ethanol 5 bis 30 Gew.-% ist, und
ein Mischungsanteil von Natriumlactat 1 bis 30 Gew.-% ist.

2. Das Virusinaktivierungsmittel nach Anspruch 1, wobei ein mittlerer Partikeldurchmesser von Calciumhydroxid, Calciumoxid und calciniertem Calcium 0,1 bis 10 µm ist.

## Revendications

1. Utilisation non thérapeutique d'un agent pour inactiver un norovirus et le virus de la grippe A, dans laquelle l'agent est
dans une solution aqueuse ou une dispersion aqueuse
préparée par mélange dans de l'hydroxyde de calcium, de l'oxyde de calcium et/ou du calcium calciné et
par mélange en outre dans de l'éthanol et du lactate de sodium,
dans laquelle une proportion totale de mélange des composants mélangés parmi l'hydroxyde de calcium, l'oxyde de calcium et le calcium calciné est de 0,01 à 30 % en poids,
une proportion de mélange de l'éthanol est de 5 à 30 % en poids, et
une proportion de mélange du lactate de sodium est de 1 à 30 % en poids.

2. Agent d'inactivation de virus selon la revendication 1. dans lequel un diamètre moyen de particule d'hydroxyde de calcium, d'oxyde de calcium et du calcium calciné est de 0,1 à 10 µm.
